# FASCICULE DE BREVET EUROPEEN

(11) **EP 2 464 637 B1**
(45) Date de publication et mention de la délivrance du brevet: **18.04.2018**
(21) Numéro de dépôt: 10743104.1
(22) Date de dépôt: 10.08.2010
(51) Int. Cl.: C07D 327/04, C07K 5/10, A61K 51/04, A61K 51/08, C07K 1/13

(54) **DERIVÉS DE COMPOSÉS À BASE DE SULTONES SUBSTITUÉS PAR DES NUCLÉOPHILES, NOTAMMENT DES RADIONUCLÉIDES, ET LEUR UTILISATION POUR LE MARQUAGE DE MACROMOLECULES**
NUKLEOPHIL-SUBSTITUIERTE SULTONVERBINDUNGSDERIVATE, IM BESONDEREN DURCH RADIONUKLIDE, UND VERWENDUNG ZUR MARKIERUNG VON MAKROMOLEKÜLEN
SULTONE COMPOUND DERIVATIVES SUBSTITUTED BY NUCLEOPHILES, IN PARTICULAR RADIONUCLIDES, AND USE THEREOF FOR MARKING MACROMOLECULES

(30) Priorité: 11.08.2009 FR 0955610
(43) Date de publication de la demande: 20.06.2012
(73) Titulaire: Advanced Accelerator Applications, 01630 Saint Genis Pouilly (FR)
(72) Inventeur: BOUTEILLER, Cédric, F-74520 Vulbens (FR)
(74) Mandataire: Cabinet Plasseraud
(86) Numéro de dépôt international: PCT/EP2010/061646
(87) Numéro de publication internationale: WO 2011/018467

(56) Documents cités:
- US-A- 4 064 167
- US-A- 4 331 760
- BROWN, JOSEPH P. ET AL: "Flavonoid sweeteners. Synthesis and intestinal absorption of selected sulfoalkylated hesperetin-3-14C derivatives in the rat" JOURNAL OF AGRICULTURAL AND FOOD CHEMISTRY , 26(6), 1418-22 CODEN: JAFCAU; ISSN: 0021-8561, 1978, XP002600959
- BUREEVA S ET AL: "Inhibition of classical pathway of complement activation with negative charged derivatives of bisphenol A and bisphenol disulphates" BIOORGANIC & MEDICINAL CHEMISTRY, PERGAMON, GB, vol. 13, no. 4, 15 février 2005 (2005-02-15), pages 1045-1052, XP004776007 ISSN: 0968-0896
- IKEDA, KEN ET AL: "Methanesulfonic acid derivative of drugs. IV. Methanesulfonic acid derivative of p,p'-diaminodiphenylsulfone. I. Hydrolysis rate in vitro" CHEMICAL & PHARMACEUTICAL BULLETIN , 21(6), 1198-204 CODEN: CPBTAL; ISSN: 0009-2363, 1973, XP002575503

## Description

### Domaine de l'invention

Le domaine de l'invention est celui des composés chimiques bioactifs complexes de hauts poids moléculaires (macromolécules), tels que les peptides, protéines et oligonucléotides, utilisables comme précurseurs de (radio)pharmaceutiques.

L'invention vise particulièrement les stratégies de marquage et les procédés d'obtention de ces macromolécules ainsi que leur utilisation, de préférence une fois radiomarquées, en thérapeutique (médecine nucléaire *i.a*) et/ou en diagnostic (imagerie nucléaire *i.a*) selon le radioélément.

### Art antérieur

Les macromolécules sont de plus en plus proposées comme composés (radio)pharmaceutiques et leurs applications semblent très prometteuses particulièrement en médecine nucléaire et oncologie. Une revue récente¹ sur le marquage au fluor-18 de macromolécules cite une étude qui pronostique que les peptides représenteront à eux seuls environ 50% des médicaments en stade clinique dans les prochaines années. Ces peptides sont également des traceurs radioactifs potentiels pour l'imagerie médicale, notamment pour la Tomographie par Emission de Positons (TEP) ou la Tomographie par Emission.

Dans le cas particulier de la TEP, il y a actuellement peu d'exemples de macromolécules marquées au fluor-18. Les marquages radioactifs des protéines sont jusqu'alors principalement effectués avec des métaux radioactifs par complexation (^{99m}Tc, ⁶⁸Ga, ¹¹¹In). Les avantages du fluor-18 comme radioisotope pour la TEP liés au développement des installations de cyclotron en France et dans le monde entier, font que le développement de méthodes de marquage au fluor-18 de ces macromolécules suscite désormais un intérêt indéniable.

Les réactions de radiofluoration avec le fluor-18 dépendent du précurseur marqué obtenu lors de la production de l'atome radioactif. Le fluor-18 peut être obtenu sous sa forme électrophile [¹⁸F]-F₂ (employé dans des réactions électrophiles sous cette forme ou sous forme d'hypofluorite moins réactif^{2,3} [¹⁸F]-AcOF) ou sous sa forme nucléophile [¹⁸F]-F⁻ (employé dans des réactions nucléophiles).

La réaction de fluoration électrophile est délicate à mettre en oeuvre et connaît de nombreuses limitations. Par cette voie de radiosynthése, le rendement radiochimique des produits fluorés est limité à 50% (à partir de [¹⁸F]-F₂, seulement un des deux atomes de fluor est marqué et est incorporé dans le réactif ou le produit) et la radioactivité spécifique finale est inférieure à 20 mCi/µmol (0,74 GBq/µmol). Cette méthode de radiosynthèse n'est donc pas adaptée à des traceurs destinés à des études de quantification et de distribution de récepteurs. Elle est la méthode qui a été initialement utilisée pour préparer le [¹⁸F]-FDG et qui reste utilisée pour préparer la [¹⁸F]-Fluoro-L-DOP⁴.

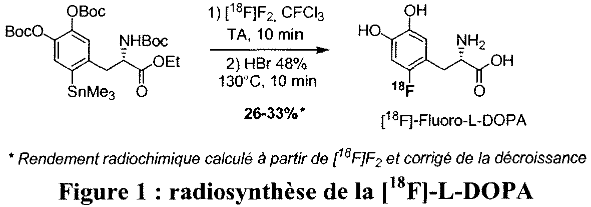

Ogawa et coll.⁵ ont décrit le marquage au fluor-18 du pentapeptide cyclique cyclo(RGDfMeV) ci-dessous par réaction électrophile directe avec l'hypofluorite [¹⁸F]-AcOF du noyau phényle du motif tyrosine.

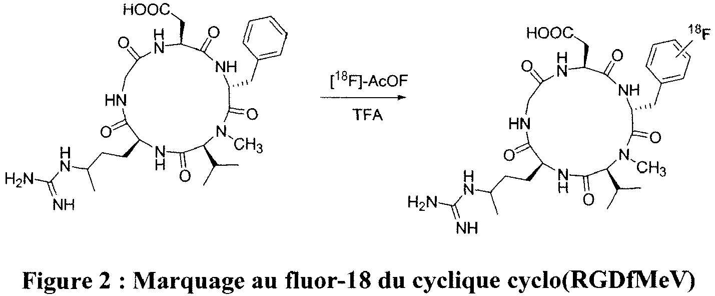

Cependant, en plus des limites de la voie électrophile citées précédemment (faible rendement radiochimique, faible radioactivité spécifique), cette synthèse présente l'inconvénient de conduire à une multitude de composés (régioisomères, composés mono- ou di- fluorés) qui ne peuvent pas être séparés. Par ailleurs, la méthionine, la cystéine et le tryptophane pouvant être oxydés par l'hypofluorite, cette méthode n'est pas applicable au marquage de peptides contenant ces acides aminés sensibles à l'oxydation. L'ion [¹⁸F]-F⁻produit par le cyclotron est récupéré en solution aqueuse. Tel quel, il offre très peu de possibilités synthétiques.⁶ En raison de sa forte énergie de solvatation (> 100 kJ.mol⁻¹), il est très peu nucléophile en présence d'eau. Afin d'augmenter son caractère nucléophile, il est transformé en sel anhydre de gros cation (césium, rubidium, tétraalkylammonium) ou mis en présence de cryptant lorsque le contre-ion est le potassium⁷ et séché. La réaction de substitution nucléophile est effectuée dans des solvants fortement dissociants (polaires aprotiques) comme le DiMéthylFormaldéhyde (DMF), le DiMéthylSulfOxyde (DMSO) ou l'acétonitrile. La plupart des composés radiopharmaceutiques marqués au fluor-18 ont été synthétisés par substitution nucléophile principalement en série aliphatique et série *homo*aromatique. L'exemple le plus connu est la radiosynthèse du [¹⁸F]-FDG⁸.

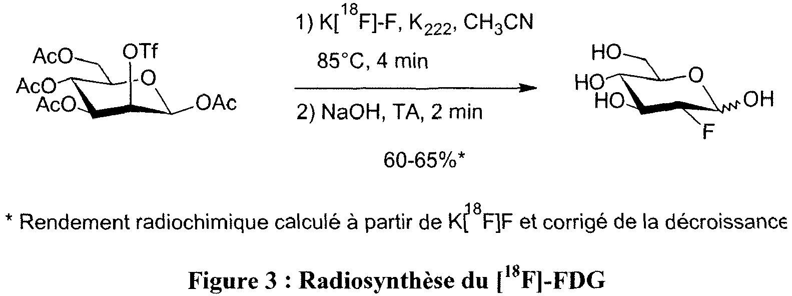

Les peptides et protéines comportent de nombreux protons labiles (hydroxyles, amides, amines, thiols, acides) pour lesquels une protection n'est pas toujours envisageable, rendant le marquage direct de ces macromolécules par substitution nucléophile le plus souvent inenvisageable.

L'introduction d'un fluor-18 dans une macromolécule de nature peptidique est le plus souvent réalisée via un groupe prosthétique portant le radioisotope¹. Cette approche implique alors la préparation du groupement prosthétique fonctionnalisé et radiomarqué suivie de sa conjugaison avec une fonction réactive du peptide ou de la protéine. Cette stratégie a l'avantage de permettre d'utiliser des conditions drastiques pour la préparation de l'entité prosthétique radiomarquée, la conjugaison de ce dernier à la macromolécule pouvant être ensuite réalisée dans des conditions douces pour préserver son intégrité.

Un certain nombre de groupements prosthétiques marqués au fluor-18 sont décrits dans la littérature. Ils peuvent être classés selon leur fonction réactive et le type de réaction par laquelle ils seront couplés à la macromolécule (amines, hydrazines, oximes, acides, aldéhydes...). La plupart d'entre eux sont conçus pour être couplés directement à un peptide ou une protéine *via* une fonction amine d'un résidu aminoacide (*N*-terminal α-NH₂ ou interne ε-NH₂ d'une lysine) ou éventuellement *via* un espaceur alkylamino. Ils sont caractérisés par une fonction acide carboxylique ([¹⁸F]-AFB) ou ester activé (ester de N-hydrosuccinimide [¹⁸F]-SFB ou de nitrophényle).

Tous ces groupements prosthétiques radiomarqués se différencient par leur accessibilité en radiosynthèse (nature et facilité de synthèse du précurseur de radiomarquage, efficacité de l'étape de fluoration, nombre total d'étapes de la radiosynthèse, rendement radiochimique global, purification), leur efficacité dans la réaction de conjugaison et leur stabilité *in vivo.*

En plus des contraintes relatives à la chimie du fluor-18, les fabricants de ces composés radiomarqués sont confrontés aux contraintes relatives à l'automatisation de l'ensemble de la synthèse de ces composés. En effet, les normes pharmaceutiques (GMP) tout autant que la radioprotection rendent impossible la réalisation de ces synthèses sans automates. Tout ceci milite en faveur de la limitation du nombre d'étapes et les facilités de purification dans ce type de synthèse.

Par ailleurs, Brown et al (J. Agric. Food Chem., 1978, Vol 26 pp 1418-1422) ont décrit l'utilisation de composés sulfoalkylés marqués au ¹⁴C et leurs utilisations dans des études d'absorption intestinale. Le brevet US4,331,760 décrit des agents diagnostics dérivés de la sulfonphtaléine et comprenant un radical qui peut être un acide aminé ou un peptide ayant un groupement protecteur azote (voir revendication 1). Le brevet US4,064,167 décrit des oligomères de dihydrochalcones utiles comme édulcorant non-nutritif dans des produits alimentaires.

### Objectifs et problème technique

Compte tenu de cet art antérieur, la présente invention vise à remédier aux inconvénients de l'art antérieur et à satisfaire au moins l'un des objectifs essentiels suivants :
→ Fournir de nouveaux groupements prosthétiques de synthèse simple, aisée et automatisable et susceptibles de constituer des précurseurs de marquage de macromolécules pour le développement de (radio)pharmaceutiques économiques et performants.
→ Fournir de nouveaux groupements prosthétiques de synthèse simple, aisée et automatisable et susceptibles de constituer des précurseurs universels de marquage de macromolécules pour le développement de (radio)pharmaceutiques selon une même voie de synthèse.
→ Fournir de nouveaux groupements prosthétiques de synthèse simple, aisée et automatisable et susceptibles de constituer de nouveaux précurseurs de marquage de macromolécules pour le développement de (radio)pharmaceutiques et ceci quel que soit le radionucléide concerné, e.g. l'iode, le chlore ou encore le fluor.
→ Fournir un nouveau procédé de marquage de macromolécules via le couplage avec un groupement prosthétique d'une nouvelle famille pour le développement de (radio)pharmaceutiques, simple, aisé et automatisable, ledit procédé offrant au moins l'un des avantages suivants :
   ∘ une réduction d'étapes de synthèse (par exemple 2 vs 3-5 pour les autres méthodes de marquage via d'autres groupements notamment prosthétiques),
   ∘ une augmentation des rendements (radio)chimiques à très faible température et en des temps très courts,
   ∘ des facilités en termes de séparation des composés intermédiaires et finaux,
   ∘ pas de production de produits secondaires,
   ∘ adaptation au marquage de nombreuses macromolécules,
   ∘ possibilité de réaliser le couplage du (ou des) radionucléide(s) avec la macromolécule dans l'eau.
→ Fournir de nouveaux médicaments et/ou traceurs radioactifs performants et économiques, à partir de cette nouvelle méthode de marquage de macromolécule.
→ Fournir une nouvelle utilisation de dérivés de sultones pour le marquage de macromolécules avec un radionucléide nucléophile.
→ Fournir une nouvelle utilisation de sultones comme entité de liaison entre deux nucléophiles ou structures portant un nucléophile, par ouverture des cycles sultone.

### Description succincte de l'invention

Ces objectifs, parmi d'autres, sont satisfaits par l'invention qui concerne dans un premier aspect l'utilisation de composés dérivés de précurseurs de type di-sultones, de préférence polysultones pour le marquage de macromolécules avec un radionucléide nucléophile. Ces composés sont caractérisés en ce qu'ils sont chacun obtenus par substitution d'un précurseur qui comprend au moins deux sultones reliées l'une à l'autre par au moins un espaceur et dont la formule (1) est la suivante : 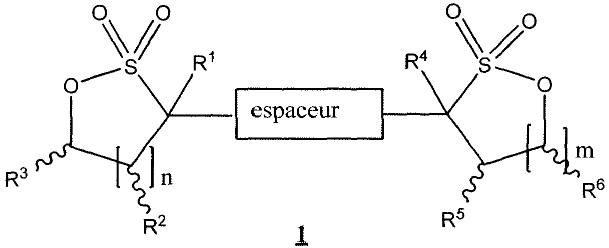 dans laquelle
- les groupements R¹ à R⁶, identiques ou différents, correspondent à l'hydrogène ou un alkyle, un aryle, un arylalkyle, un alkylaryle, un acyle, un cycloalkyle; R³ à R⁶ correspondant de préférence à l'hydrogène ;
- n, m est un entier, de préférence compris entre 0 et 2 ;
- l'espaceur est une structure divalente, de préférence choisie parmi celles comportant un ou plusieurs groupements aromatiques, alkyles, alcènes, alcynes, éventuellement combinés entre eux ;
par ouverture d'au moins l'un des cycles sultones par au moins un radical nucléophile et de préférence par au moins un (de préférence un) radical nucléophile R¹ sur chacun des cycles sultones ouverts.

De préférences, les composés sont des composés de formule générale **2** ou **3** : 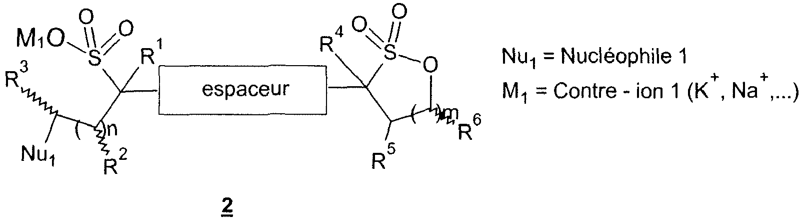 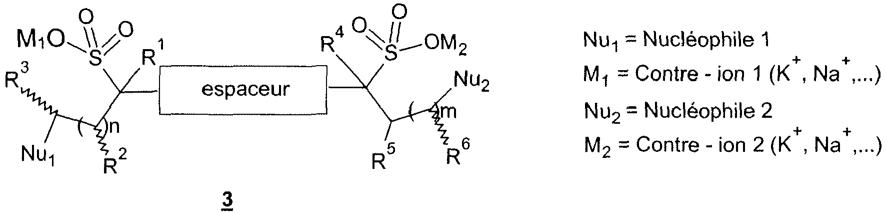 formules dans lesquelles :
- Nu₁, Nu₂ des nucléophiles identiques ou différents entre eux ;
- M₁, M₂ sont des contre-anions identiques ou différents entre eux et choisi parmi les cations, de préférence dans le groupe des cations monovalents, par exemple K⁺, Na⁺.

Dans un deuxième aspect, l'invention a pour objet l'utilisation de précurseurs **1** comprenant chacun au moins deux sultones reliées l'une à l'autre par au moins un espaceur caractérisés en ce qu'ils répondent à l'une des formules suivantes :

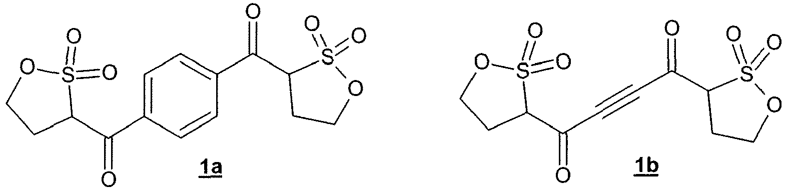

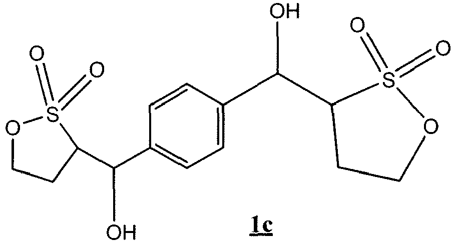

Un sous-groupe particulier des précurseurs **1** comprend :

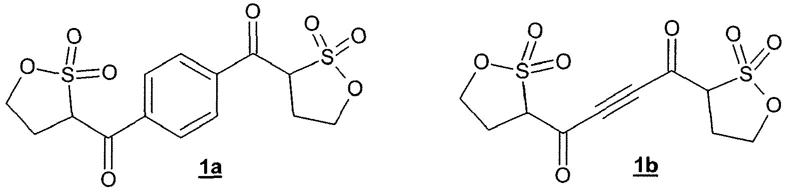

Dans un troisième aspect, l'invention a pour objet un procédé de synthèse de précurseurs **1** comprenant chacun au moins deux sultones reliées l'une à l'autre par au moins un espaceur, caractérisé en ce qu'il consiste essentiellement à faire réagir des sultones - avantageusement des butanes sultones, des propanes sultones et/ou des éthanes sultones- et un polyester, de préférence selon un mécanisme de lithiation de chaque sultone en alpha du soufre suivie d'une réaction avec le polyester électrophile qui est pour ce faire ajouté au milieu réactionnel.

Dans un quatrième aspect, l'invention a pour objet un procédé de synthèse des composés dérivés de précurseurs **1** polysultones caractérisé en ce qu'il comprend les étapes suivantes :
a. Mise en oeuvre ou synthèse d'une polysultone, de préférence une double sultone, comprenant chacune au moins deux sultones reliées l'une à l'autre par au moins un espaceur ;
b. Ouverture de l'une des sultones avec un premier nucléophile conduisant à la formation d'un sulfonate ;
c. Séparation de la polysultone (de préférence la double sultone) précurseur du sulfonate formé par différence de polarité ;
d. Recueil du sulfonate formé ;
e. Couplage du sulfonate formé avec au moins un second (de préférence un) nucléophile par l'ouverture d'une (de préférence de la) seconde sultone ;
f. Recueil de la polysulfonate (de préférence disulfonate) obtenu à l'étape e.

Dans un cinquième aspect, l'invention vise un médicament ou un produit de diagnostic (traceur) comprenant au moins un composé selon l'invention ou obtenu par l'un des procédés selon l'invention.

Dans un sixième aspect, l'invention vise l'utilisation des composés selon l'invention ou obtenus par le procédé selon l'invention pour le marquage de macromolécules avec radionucléide nucléophile, ou
comme entité de liaison entre deux nucléophiles ou structures portant un nucléophile, par ouverture des cycles sultone.

Les principaux avantages de l'invention sont les suivants :
- Utilisation de la même approche pour marquer quel que soit le radionucléide ;
- Réduction du nombre d'étapes de synthèse ;
- Obtention de rendements radiochimiques très élevés à très faible température et en des temps très courts ;
- Possibilité de séparation du précurseur de départ (très apolaire) du produit formé (très polaire) ;
- Pas de production de produits secondaires ;
- Possibilité d'automatisation ;
- Adaptation au marquage de nombreuses macromolécules ;
- Couplage avec la macromolécule possible dans l'eau ;
- Simplicité ;
- Economie ;
- Accès à de nouveaux composés ouvrant de multiples développements en thérapeutique et diagnostic.

### Description détaillée de l'invention

Dans les formules générales des nouveaux composés **1, 2**, **3**, on se réfère aux définitions suivantes :
"*alkyle*" correspond par exemple à un groupement alkyle monovalent saturé, ou linéaire ramifié, en C1-C30, de préférence C1-C20, et, plus préférentiellement encore en C1-C10. Des exemples de groupes alkyle sont notamment méthyle, éthyle, isopropyle, n-propyle, tert-butyle, isobutyle, n-butyle, n-pentyle, isoamyle et 1,1-diméthylpropyle.
"*aryle*" correspond par exemple à un ou plusieurs groupements monovalent(s) aromatiques condensés ou non, ayant de 6 à 18 atomes de carbone, monocyclique ou polycyclique et de préférence monocyclique ou bicyclique. Il doit être entendu que, dans le cadre de l'invention, par radical aromatique polycyclique, on entend un radical présentant deux ou plusieurs noyaux aromatiques, condensés (orthocondensés ou ortho et péricondensés) les uns aux autres, c'est-à-dire présentant, deux à deux, au moins deux carbones en commun. A titre d'exemple d'aryle, on peut mentionner les radicaux phényle, naphtyle, anthryle et phénanthryle.
"*arylalkyle*" correspond par exemple un groupe alkyle tel que défini ci-dessus, substitué par un ou plusieurs groupes aryle sur sa chaîne hydrocarbonée, le groupe aryle étant tel que défini ci-dessus. Des exemples en sont benzyle et triphénylméthyle.
"*alkylaryle*" correspond par exemple à alkyle monovalent substitué ou relié à un ou plusieurs groupements aromatiques monovalent(s), éventuellement substitué.

Par "*acyle*", on entend un groupe Rₒ-CO- où Rₒ représente alkyle tel que défini ci-dessus ; ou bien un groupe Ar-CO- où Ar représente un groupe aryle tel que défini ci-dessus, ou bien arylalkyle dans lequel aryle et alkyle sont tels que définis ci-dessus et dans lequel la partie aryle est éventuellement substituée e.g. par alkyle.

Par "*cycloalkyle*", on entend un radical hydrocarboné saturé mono- ou polycyclique, de préférence mono- ou bicyclique, présentant préférablement de 3 à 10 atomes de carbone, mieux encore de 3 à 8. Par radical hydrocarboné saturé polycyclique, on entend un radical présentant deux ou plusieurs noyaux cycliques rattachés les uns aux autres par des liaisons σ ou/et condensés deux à deux. Des exemples de groupes cycloalkyle polycycliques sont adamantane et norbornane. Des exemples de groupes cycloalkyle monocycliques sont cyclopropyle, cyclobutyle, cyclopentyle, cyclohexyle, cycloheptyle et cyclooctyle.

Par "*alcène*" ou "*alcényle*", on entend e.g. une chaîne hydrocarbonée insaturée, linéaire ou ramifiée, présentant au moins une double liaison oléfinique, et plus préférablement une seule double liaison. De préférence, le groupe alcényle présente de 2 à 8 atomes de carbone, mieux encore de 2 à 6. Des exemples préférés de groupes alcényle sont les groupes allyle et homoallyle.

Par "*alcyne*" "*alcynyle*", on entend e.g. selon l'invention, une chaîne hydrocarbonée insaturée, linéaire ou ramifiée, présentant au moins une triple liaison acétylénique, et plus préférablement une seule triple liaison. De préference, le groupe alcynyle présente de 2 à 8 atomes de carbone, mieux encore de 2 à 6 atomes de carbone. A titre d'exemple, on peut citer le groupe acétylényle, ainsi que le groupe propargyle.

L'espaceur peut par exemple être une structure aromatique (fonctionnalisée ou non), une structure alcyne ou tout autre structure. Dans les cas les plus simples, les groupements R¹-R⁶ seront des protons (en particulier sur les positions d'attaque des nucléophiles R⁶ et R³) mais ils peuvent également être tout autre groupement.

Les précurseurs **1** selon l'invention peuvent être des disultones de formules suivantes :

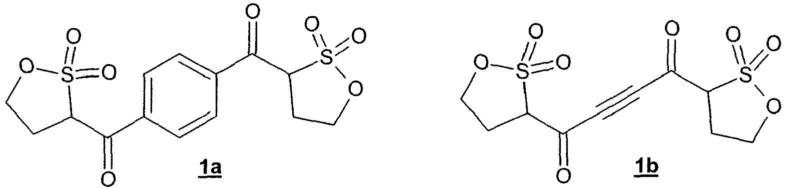

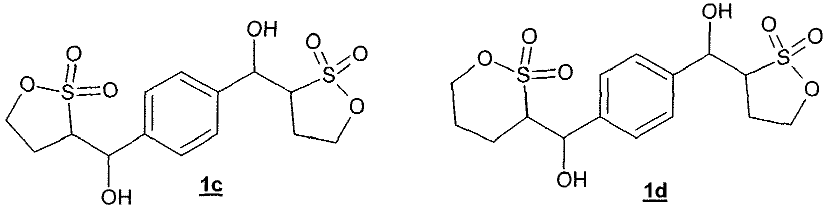

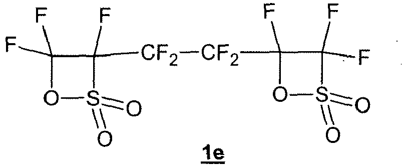

La structure **1a** correspond au cas où l'espaceur est un cycle aromatique relié aux deux sultones par deux fonctions cétone en para, où les groupements R¹-R⁶ sont tous des hydrogènes et où n = m = 1.

La structure **1b** correspond au cas où l'espaceur est un alcyne relié aux deux sultones par deux fonctions cétone, où les groupements R¹-R⁶ sont tous des hydrogènes et où n = m = 1.

La structure **1c** correspond au cas où l'espaceur est un cycle aromatique relié aux deux sultones par deux fonctions alcool, où les groupements R¹-R⁶ sont tous des hydrogènes et où n = m = 1.

La structure **1d** correspond au cas où l'espaceur est un cycle aromatique relié aux deux sultones par deux fonctions alcool en para, où les groupements R¹-R⁶ sont tous des hydrogènes et où n = 2 et m = 1.

La structure **1e** correspond au cas où l'espaceur est un motif tétrafluoroéthane reliant les deux sultones directement, où les groupements R¹-R⁶ sont tous des fluors et où n = m = 0.

### Synthèse des polysultones

Ces composés sont synthétisables à partir de produits commerciaux.

Ainsi, le procédé de synthèse du composé selon l'invention consiste essentiellement à faire réagir des sultones - avantageusement des butanes sultones, des propanes sultones et/ou des éthanes sultones- et par exemple un polyester, de préférence selon un mécanisme de lithiation de chaque sultone en alpha du soufre suivie d'une réaction avec le polyester électrophile qui est pour ce faire ajouté au milieu réactionnel.

Par exemple, deux équivalents de sultone correspondante et d'un équivalent d'un diester correspondant à l'espaceur via une réaction de lithiation de la sultone en alpha du soufre suivie de la réaction avec le diester en tant qu'électrophile.

Cette synthèse se base sur la chimie des sultones, composés cycliques analogues soufrés des lactones à 4, 5 ou 6 chaînons (figure 4).

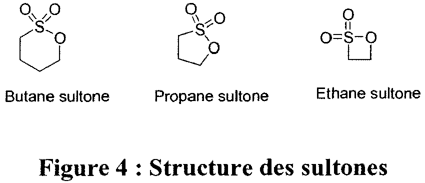

Les sultones sont bien connus pour leur réactivité avec les nucléophiles^{11,12}. Un brevet déposé en 2004 a d'ailleurs déjà fait l'objet de ce type de réactivité¹³. La réaction forme une liaison carbone-nucléophile par attaque nucléophile du carbone en alpha de l'oxygène et résulte en la formation d'une fonction sulfonate. La réactivité des sultones avec les nucléophiles tels que les cyanures, les phosphines^{14,15}, les amines^{16,17,18}, les alcoolates^{16,19} les thiolates²⁰, l'iode, le chlore et le brome,^{19,21,22} est bien connue.

Le schéma de synthèse du composé **1a** est représenté ci-dessous sur la figure 5. La lithiation en alpha du soufre de deux équivalents de propane sultone à - 78 °C dans le THF pendant 30 minutes suivie de l'ajout de 1 équivalent du diester méthylique (ou paranitrophénylique) conduit à la disultone correspondante **1a.** Les exemples qui suivent illustrent cette synthèse.

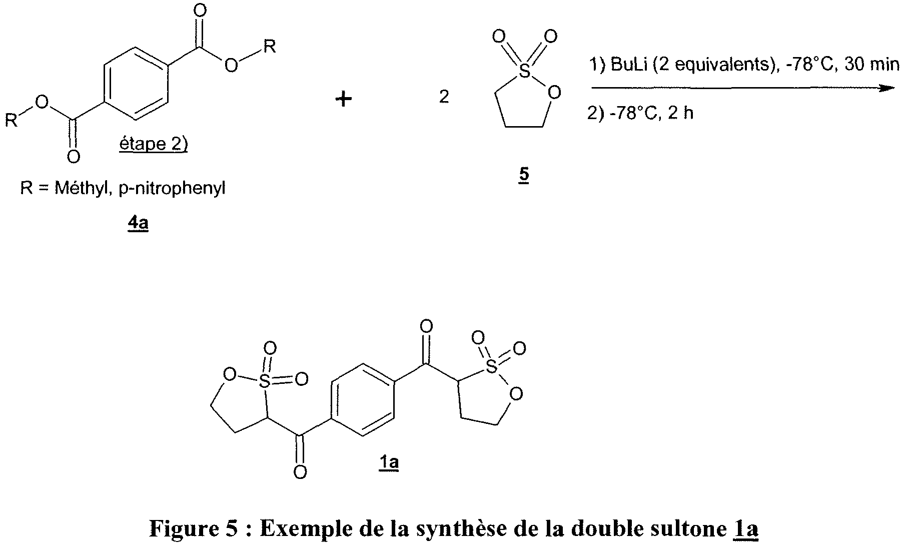

Le schéma de la synthèse du composé **1b** est représenté ci-dessous sur la figure 6. La lithiation en alpha du soufre de deux équivalents de propane sultone à -78 °C dans le THF pendant 30 minutes suivie de l'ajout de 1 équivalent du diester méthylique commercial de l'acide butynedioïque. Les exemples qui suivent illustrent cette synthèse.

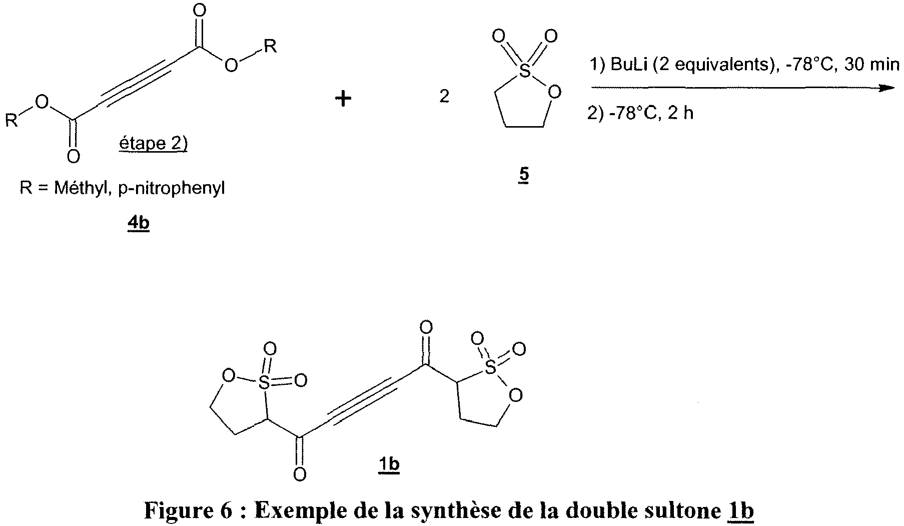

Ces polysultones, et, en particulier, les doubles sultones **1a** et **1b** peuvent par exemple être utilisées :
- soit comme "linker", entité de liaison, entre deux nucléophiles ou structures portant un nucléophile par ouverture des cycles sultone,
ou selon une modalité préférée mais non limitative de l'invention :
- pour marquer des macromolécules (portant le nucléophile ouvrant la seconde sultone) avec un radionucléide nucléophile (ouvrant la première sultone) tels que par exemple le fluor-18, le brome-76, l'iode-123, l'iode-131 ou tout autre radionucléide nucléophile ainsi que toutes les combinaisons de radionucléides nucléophiles.

Les précurseurs **1** polysultones sus-définis donnent accès à de nouveaux composés substitués chacun par au moins un radical nucléophile par ouverture d'au moins l'un des cycles sultones, de préférence par au moins un (de préférence un) radical nucléophile R¹ sur chacun des cycles sultones ouverts. Avantageusement, le(les) radical(aux) nucléophile(s) Rⁱ est(sont) un(des) radionucléide(s) R*, de préférence choisi(s) dans le groupe comprenant le fluor-18, le brome-76, l'iode-123, l'iode-131 ou tout autre radionucléide nucléophile.

De préférence, le(les) radical(aux) nucléophile(s) Rⁱ est(sont) un(des) radical(aux) actif(s) R^{a} choisi(s) dans le groupe comprenant les peptides, les protéines, les oligonucléotides, les polynucléotides ou toute autre macromolécule.

Plus préférentiellement encore, à titre d'exemple à partir de **1a** et **1b** ces composés répondent aux formules **6a** ou **6b** suivantes :

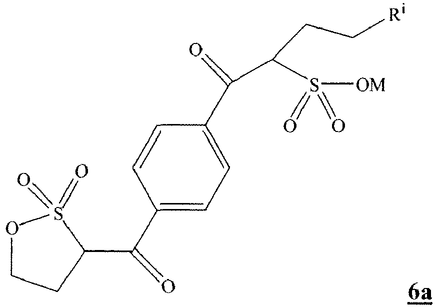

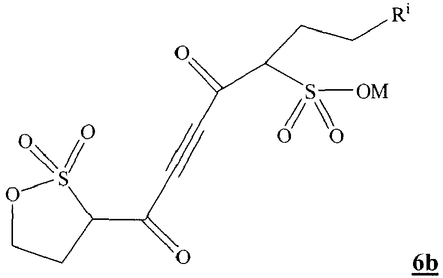

Les composés plus particulièrement préférés selon l'invention, sont des composés présentant au moins une substitution par un groupe radioactif et au moins une substitution par un groupe actif de type macromolécule (par exemple en thérapeutique ou en diagnostic). Chacun de ces composés est caractérisé en ce qu'il est substitué par un(des) radionucléide(s), de préférence choisi(s) dans le groupe comprenant le fluor-18, le brome-76, l'iode-123, l'iode-131 ou tout autre radionucléide nucléophile, par ouverture de l'un des cycles sultones et en ce qu'il est substitué par un radical nucléophile choisi dans le groupe comprenant les peptides, les protéines, les oligonucléotides, les polynucléotides ou tout autre macromolécule par ouverture de l'autre cycle sultone éventuellement via l'attaque d'une amine libre.

Plus précisément, à titre d'exemple à partir de **1a** et **1b** ces composés préférés répondent e.g. respectivement aux formules **7a** ou **7b** suivantes :

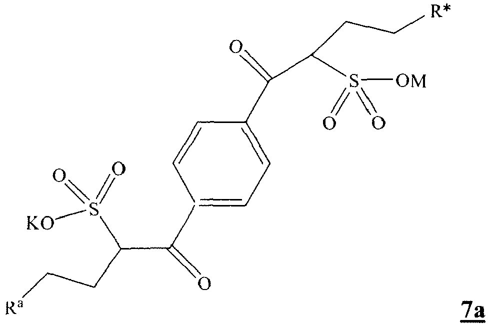

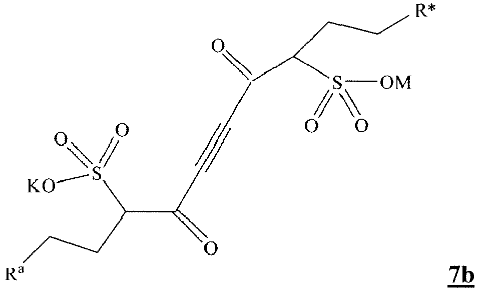

### Obtention des composés selon l'invention

Conformément à l'invention, le procédé d'obtention d'un nouveau composé issu d'un précurseur poly(e.g. double)sultone est caractérisé en ce qu'il comprend les étapes suivantes :
a. Mise en oeuvre ou synthèse d'une polysultone, de préférence une double sultone, par le procédé selon la revendication 10 ;
b. Ouverture de l'une des sultones avec un premier nucléophile conduisant à la formation d'un sulfonate ;
c. Séparation de la polysultone (de préférence la double sultone) précurseur et du sulfonate formé par différence de polarité ;
d. Recueil du sulfonate formé ;
e. Couplage du sulfonate formé avec au moins un second nucléophile par l'ouverture d'une (de préférence de la) seconde sultone ;
f. Recueil du polysulfonate (de préférence disulfonate) obtenu à l'étape e.

A titre d'exemple, le marquage de macromolécules au fluor-18 est représenté sur la figure 7 ci-dessous. Le produit **1a** est radiofluoré dans des conditions classiques de radiofluoration et le fluorosulfonate (très polaire) résultant **8** est aisément séparé de la disultone (apolaire) précurseur **1a** à travers une cartouche C18 grâce à leurs différences de polarité. La partie expérimentale de la synthèse de la référence non radioactive [¹⁹F]-**8** et de la radiosynthèse du produit [¹⁸F]-**8** est décrite dans les exemples qui suivent.

Le fluorosulfonate obtenu **8** est ensuite couplé dans l'eau (solubilité du fluorosulfonate en milieu aqueux) avec un peptide par l'ouverture de la seconde sultone avec une amine libre portée par le peptide conduisant au peptide radiofluoré **9**.

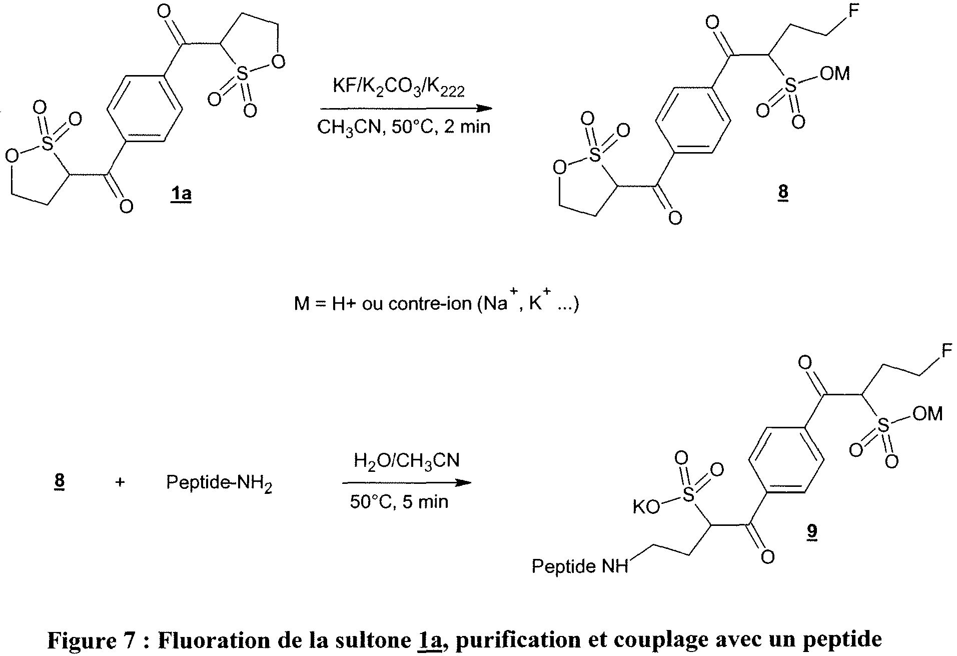

A titre d'exemple, l'utilisation de la Boc-Lysine-NH₂ **10** permet d'obtenir la Boc lysine radiofluoré **11**: Figure 7. Le mode opératoire de la synthèse de [¹⁹F]-**11** et de la radiosynthèse de [¹⁸F]-**11** sont décrits dans les exemples qui suivent.

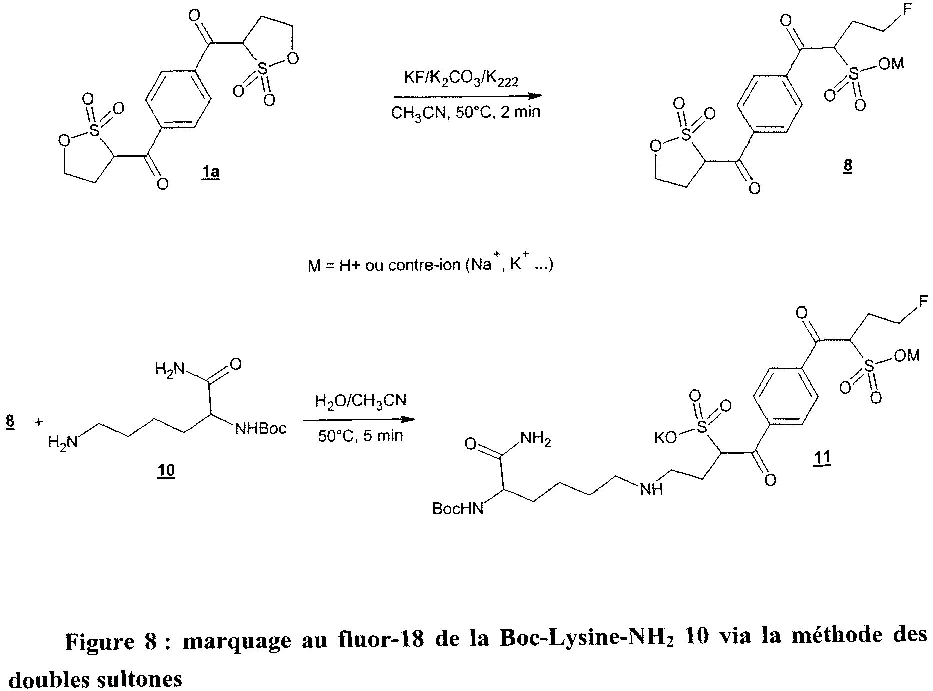

Cette stratégie de marquage peut également être étendue au marquage de la Boc-Lysine-NH₂ **10** à l'iode. L'analogue iodé **12** est obtenu par couplage de la Boc-lysine **10** avec la sultone iodée **13,** elle-même obtenue par radioiodation de la disultone **1a.** (voir figure 9 ci-dessous). Le mode opératoire de la synthèse de **13** en chimie non radioactive est décrit en annexe dans les exemples qui suivent.

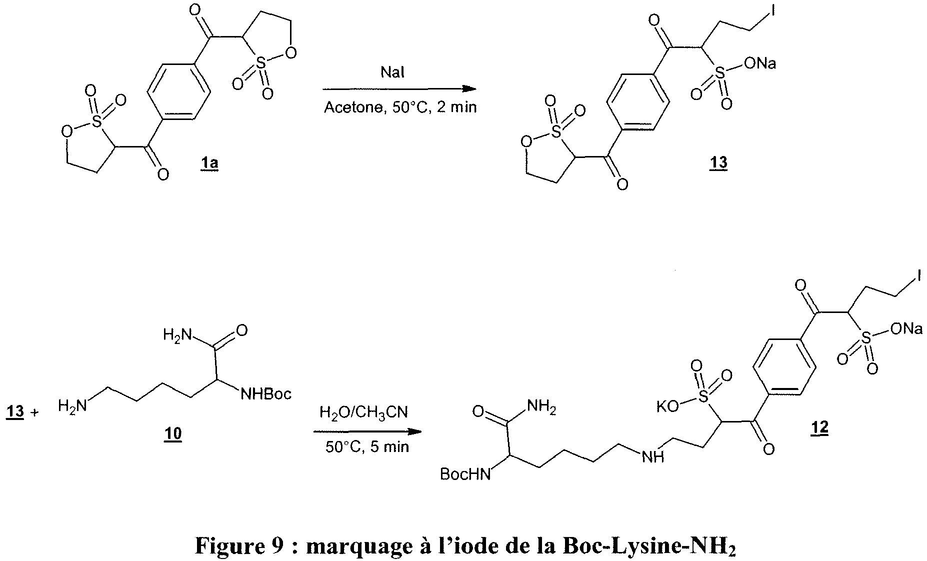

De la même façon, l'utilisation du peptide aminé RGD **14** comme précurseur de marquage permet d'obtenir le peptide radiofluoré **15 :** Figure 10. 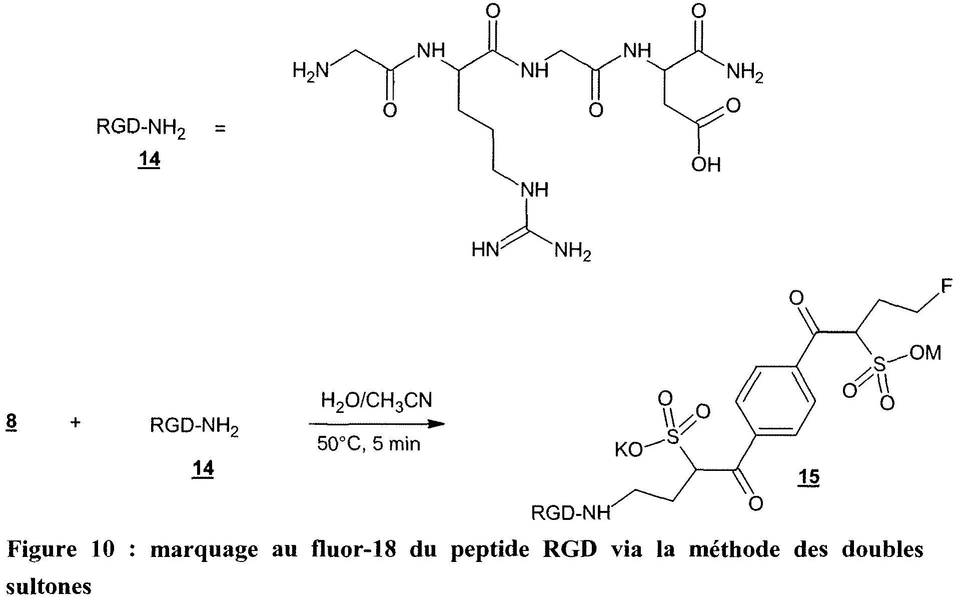 Cette stratégie de marquage peut également être étendue au marquage du peptide RGD **14** à l'iode. L'analogue iodé **16** est obtenu par couplage du peptide précurseur **14** avec la sultone iodée **13,** elle-même obtenue par radioiodation de la disultone **1a.** Le mode opératoire de la synthèse de **13** en chimie non radioactive est décrit en annexe dans la partie expérimentale.

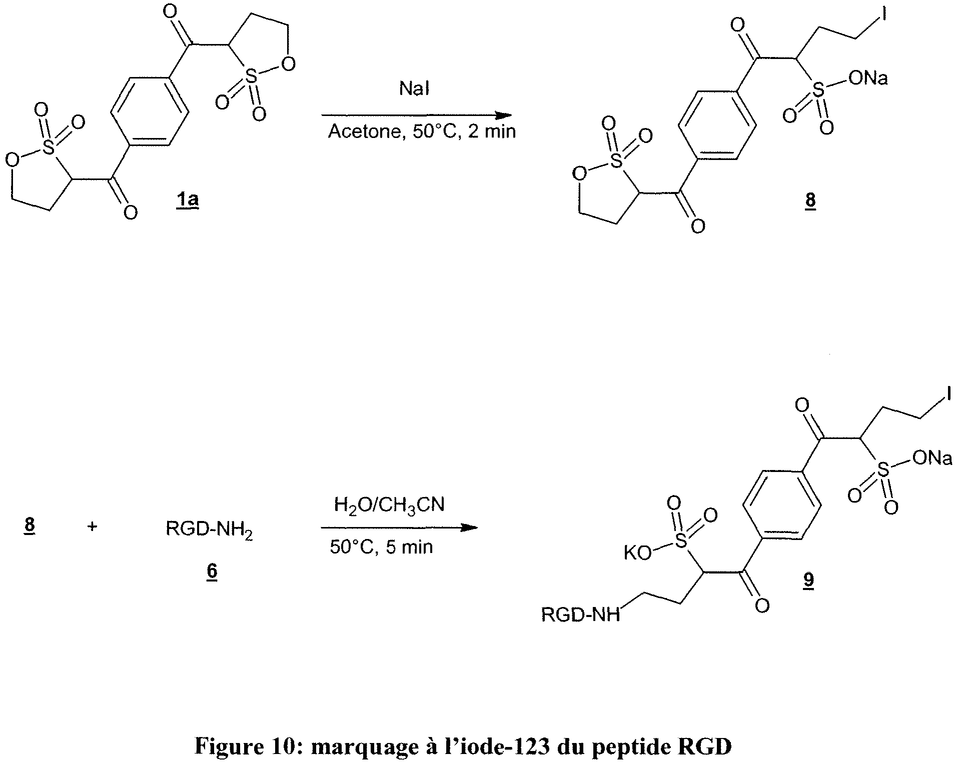

Cette stratégie de marquage permettra donc de disposer d'une seule et même méthode pour marquer, par exemple, un peptide à l'iode-123 (pour l'utiliser en TEMP) ou au fluor-18 (pour l'utiliser en TEP).

### EXEMPLES

### Méthodes CLHP

*Système A* : CLHP analytique (Hypersil gold C₁₈ colonne, 5 *µ*m, 4.6 x 100 mm) avec comme éluants CH₃CN et 0.1% d'acide trifluoroacétique dans l'eau (0.1%, v/v, pH 2.0) [100% TFA (5 min), puis gradient linéaire de 0% à 80% (40 min) en CH₃CN] à un débit de 1 mL/min. Les détections UV sont effectuées à 265 et 254 nm.
*Système B* : CLHP semi-préparative (Hypersil gold C₁₈ colonne, 5 *µ*m, 21.2 x 250 mm) avec comme éluants CH₃CN et 0.1% d'acide trifluoroacétique dans l'eau (0.1%, v/v, pH 2.0) [100% TFA (10 min), puis gradient linéaire de 0% à 5% en CH₃CN (2 min), puis gradient linéaire de 5% à 70% en CH₃CN (65 min)] à un débit de 1 mL/min. Les détections UV sont effectuées à 265 et 254 nm.
*Système C* : CLHP analytique (Hypersil gold C₁₈ column, 5 *µ*m, 4.6 x 100 mm) avec comme éluants CH₃CN et 25 mM d'acetate de triethylammonium dans l'eau (TEAA, 25mM, pH 7.0) [100% TEAA (10 min), puis gradient linéaire de 0% à 80% (40 min) en CH₃CN] à un débit de 1 mL/min. Les détections UV sont effectuées avec le mode max plot (chaque pic est détecté à son maximum d'absorption).
*Système D* : CLHP semi-préparative (Hypersil gold C₁₈ column, 5 *µ*m, 10 x 250 mm) avec comme éluants CH₃CN et 50mM de bicarbonate de triméthylammonium aqueux (TEAB, 50mM, pH 7.5) [100% TEAB (10 min), puis gradient linéaire de 0% à 5% (2 min) en CH₃CN, puis gradient linéaire de 5% à 18% en CH₃CN (13min), puis gradient linéaire de 18% à 25% (14min) en CH₃CN, puis gradient linéaire de 25% à 31% (6 min) en CH₃CN, puis gradient linéaire de 31% à 61% (15 min) en CH₃CN] à un débit de 1 mL/min. La détection UV est effectuée à 254 and 220 nm.

### EXEMPLE 1 : Synthèse de la sultone 1a:

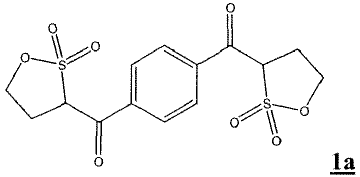

A une solution de 1,3-propane sultone (447 mg, 3.66 mmol, 2 eq) dans le THF (5 mL), sous atmosphère d'argon, à - 78 °C, est ajoutée au goutte à goutte une solution de nBuLi 1.3 M dans l'hexane (2.82 mL, 3.67 mmol, 1.99 eq). Après 1 h à - 78 °C, une solution de diméthyl téréphthalate (358 mg, 1.84 mmol, 1 eq) dans 10 mL de THF est ajoutée au goutte à goutte au mélange précédent vigoureusement agité. Le mélange est ensuite agité à - 78 °C pour 2 h 30, puis toujours à - 78 °C, la réaction est quenchée avec 1 mL d'acide acétique glacial dans 3 mL de THF (pH ∼ 6).
Le mélange est ensuite lentement radouci à TA puis dilué dans 20 mL de NaCl saturé dans l'eau et 50 mL d'acétate d'éthyle. Un solide est observé entre la phase organique et la phase aqueuse et est filtré. La phase aqueuse est lavée avec 30 mL d'acétate d'éthyl. Les phases organiques sont réunies, séchées avec du MgSO₄, filtrées et concentrées sous vide. Le produit brut obtenu est ensuite purifié par chromatographie flash sur une colonne de silice avec comme phase mobile un mélange acétone-cyclohexane (2 : 3, v/v). La bis-sultone obtenue est isolée sous forme d'un solide beige (270 mg, rendement 40%). *R*_{f} 0.42 (acétone-cyclohexane, 2 : 3, v/v);
¹H RMN (300 MHz, acetone-d₆,) : δ 8.20 (s, 4H), 5.63-5.57 (m, 2H), 4.59-4.49 (m, 4H), 3.17-3.08 (m, 2H), 2.80-2.71 (m, 2H).
¹³C RMN (75 MHz, DMSO-d₆) : δ 195.4, 140.8, 128.4, 63.3, 58.8, 32.2
CLHP (système A): *t*_{R}= 26.7 min (pureté 96.6%);
UV (enregistré pendant l'analyse CLHP): λₘₐₓ 265 nm;
MS (ESI, mode négatif): *m*/*z* 373.27 [M + H]⁻ C₁₄H₁₄O₈S₂ 374.01

### EXEMPLE 2 : Synthèse de la bis-sultone monofluorée 8 :

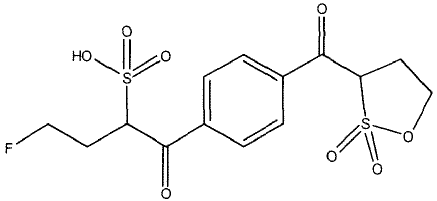

Dans un ballon sont introduits 200 µL d'une solution de fluorure de potassium dans l'eau mQ (62.2 mg/mL, 12.44 mg, 0.214 mmol, 1eq), kryptofix (95 mg, 0.252 mmol, 1.18 eq) et 1 mL d'acétonitrile. La bis-sultone **1a** (80 mg, 0.214 mmol, 1 eq) en solution dans 5,5 mL d'acétonitrile est ensuite ajoutée. Le mélange est agité à TA et l'avancement est suivi en CLHP (Système A).
Le brut réactionnel est purifié par CLHP semi-préparative. Après identification par spectrométrie de masse, la fraction contenant le produit est lyophilisée. Le produit est obtenu sous forme d'un solide beige (27.4 mg, 32.5 %).

¹H RMN (300 MHz, MeOD-d₃,) : δ 8.16-8.09 (m, 4H), 5.56-5.50 (ddd, J = 2.46, 6.6, 8,67 Hz, 1H), 5.07-5.02 (ddd, J = 0.96, 3.96, 9.42 Hz, 1H), 4.61-4.46 (m, 2H), 4.45-4.37 (m, 1H), 4.29-4.21 (m, 1H), 3.15-3.04 (m, 1H), 2.71-2.58 (m, 1H), 2.55-2.33 (m, 2H).
CLHP (système A): *t*_{R} = 18.8 (dial) -19 (dia2) min (pureté 85%);
UV (enregistrée lors de l'analyse CLHP): λₘₐₓ 265 nm;
MS (ESI, mode négatif): *m*/*z* 393.13 [M - H]⁻ C₁₄FH₁₅O₈S₂ 394.02

### EXEMPLE 3 : Synthèse de la Boc-lysine fluorée 11 :

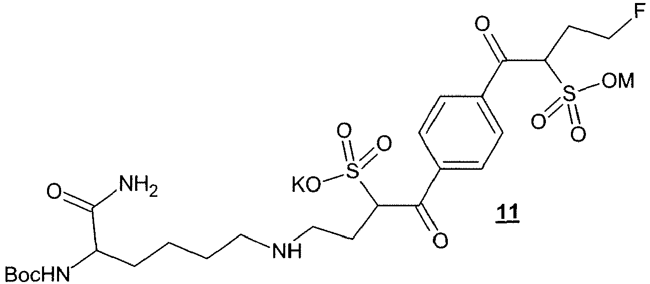

Dans une solution de la sultone fluorée **8** (31.5 mg, 0.08 mmol, 1 eq) dans le DMF (1,8 mL), à température ambiante, est ajouté la Boc-Lys-NH₂ (21.5 mg, 0.088 mmol, 1.1 eq) dans le DMF (0.8 mL). La réaction est suivie par CLHP (Système A). Une fois la double sultone consommée totalement, le brut est purifié par CLHP semi-préparative (Système B). Les analyses masse effectuées ont permis d'isoler les bonnes fractions. Un traitement sur une résine Dowex (Dowex ® 50WX8-400) est ensuite nécessaire pour reprotoner les fonctions sulfonates. Une dernière purification sur une colonne semi-préparative est ensuite nécessaire pour séparer du produit de clivage du groupement boc dû au traitement acide à travers la résine. Le produit final est obtenu sous forme d'un solide blanc (2.8 mg, 7.3%).

¹H RMN (DMSO, 300 MHz) δ 8.20-8.17 (dd, 2H), 7.82-7.77 (t, J = Hz, 2H), 4.93-4.91 (m, 1H), 4.51-4.42 (m, 1H), 4.24 (t, J = 8.6 Hz, 2H), 3.77 (t, J = 7.4 Hz, 2H), 2.67-2.60 (m, 2H), 1.77-1.74 (m, 2H), 1.65-1.55 (m, 2H), 1.37 (s, 9H), 1.31-1.24 (m, 2H).
HPLC (système C): *t*_{R}=15.8 (dial) -16 (dia2) min (pureté 97.3%);
UV (enregistré lors de l'analyse CLHP): λₘₐₓ 265 nm;
MS (ESI, negative mode): *m*/*z* 638.07 [M - H]⁻ ; 620.07 [M - H + H₂O]⁻C₂₅FH₃₈N₃O₁₁S₂ 639.19

### Bibliographie

₁ Wester H. J. et Schottelius M. Ernst.Schering.Res.Found.Workshop 2007, 79-111.
₂ Lasne M. C., Perrio C., Rouden J., Barre L., Roeda D., Dolle F., et Crouzel C. Top.Curr.Chem. 2002, 222, 201-258.
₃ welsch, M. J., Redvanly, C. S., et Editors. Handbook of Radiopharmaceuticals: Radiochemistry and Applications 2003, 848.
₄ Heiss, W.-D. et Hilker, R. Eur.J.Neurol. 2004, 11, 5-12.
₅ Ogawa, M., Hatano, K., Oishi, S., Kawasumi, Y., Fujii, N., Kawaguchi, M., Doi, R., Imamura, M., Yamamoto, M., Ajito, K., Mukai, T., Saji, H., et Ito, K. Nucl. Med. Biol. 2003, 30, 1-9.
₆ Clark, J. H. Chem.Rev. 1980, 80, 429-452.
7 Hamacher, K., Coenen, H. H., et Stoecklin, G. J.Nucl.Med. 1986, 27, 235-238.
₈ Kim H. W., Jeong J. M., Lee Y. S., Chi D. Y., Chung K. H., Lee D. S., Chung J. K., et Lee M. C. Appl.Radiat.Isot. 2004, 61, 1241-1246.
₉ DuBois, Grant E.; Crosby, Guy A. Dihydrochalcones oligomers. US (1977), US 4064167 19771220
₁₀ Kim, H.-K. et al., Proton conducting inorganic material, polymer nano-composite membrane including the
   same, and fuel cell adopting the polymer nano-composite membrane, US 2006/0269816 A1
₁₁ Deacon, T., Farrar, C. R., Sikkel, B. J., et Williams, A. J.Am.Chem.Soc. 1978, 100, 2525-2534.
₁₂ Roberts D. W. et Williams D. L. Tetrahedron 1987, 43, 1027-62.
₁₃ WO2004113391A2
₁₄ Cole A. C., Jensen J. L., Ntai I., Tran K. L., Weaver K. J., Forbes D. C., et Davis J. H., Jr. J. Am. Chem. Soc. 2002, 124, 5962-5963.
₁₅ Paetzold, E., Kinting, A., et Oehme, G. J.Prakt.Chem. 1987, 329, 725-731.
₁₆ Suga, K., Miyashige, T., Takada, K., Watanabe, S., et Moriyama, M. Aust.J.Chem. 1968, 21, 2333-2339.
₁₇ Erman, W. et Kretschmar, H. C. J.Org.Chem. 1961, 26, 4841-4850.
₁₈ Zeid, I. et Ismail, I. Justus Liebigs Annalen der Chemie 1974, 667-670.
₁₉ Helberger, J. H., Manecke, G., et Heyden, R. Justus Liebigs Annalen der Chemie 1949, 565, 23-35.
₂₀ King, J. F., Skonieczny, S., et Poole, G. A. Can.J.Chem. 1983, 61, 235-243.
₂₁ Preston A. J., Gallucci J. C., et Paquette L. A. J.Org.Chem. 2006, 71, 6573-6578.
₂₂ King, J. F. et Khemani, K. C. Can.J.Chem. 1989, 67, 2162-2172.

### Liste des abréviations

- TA: Température Ambiante
- TFA: Acide TrifluoroAcétique
- FDG: FluoroDésoxyGlucose
- AcOF: Acétyl hypofluorite (CH₃COOF)
- OTf: Triflate = OSO₂CF₃
- K₂₂₂: Cryptand 2.2.2.
- K[¹⁸F]-F: [¹⁸F] fluorure de potassium
- K₂CO₃: Carbonate de potassium
- H₂O: Eau
- CH₃CN: Acétonitrile
- RGD: Arginine Glycine Aspartate

## Revendications

1. Utilisation d'un composé pour le marquage de macromolécules avec un radionucléide nucléophile, **caractérisée en ce que** ledit composé est de formule générale **1** ou **2** : 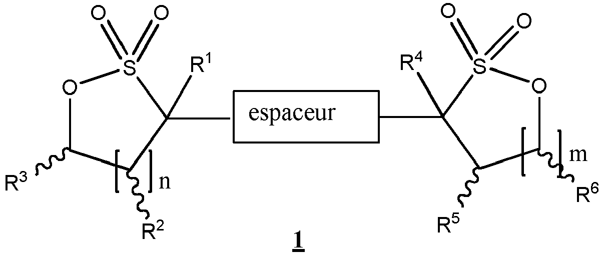 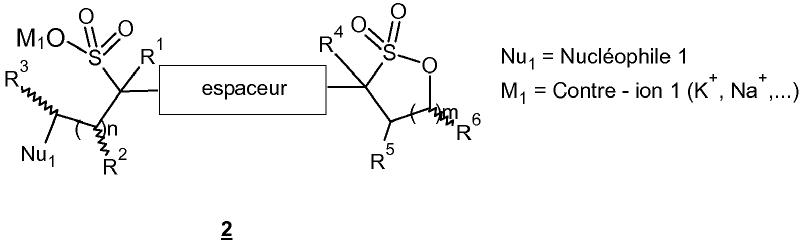 formules dans lesquelles :
• les groupements R¹ à R⁶, identiques ou différents, correspondent à l'hydrogène ou un alkyle, un aryle, un arylalkyle, un alkylaryle, un acyle, un cycloalkyle; R³ à R⁶ correspondant de préférence à l'hydrogène ;
• n, m est un entier, de préférence compris entre 0 et 2 ;
• l'espaceur est une structure divalente, de préférence choisie parmi celles comportant un ou plusieurs groupements aromatiques, alkyles, alcènes, alcynes, éventuellement combinés entre eux ;
• Nu₁ est un radionucléide nucléophile R*, de préférence choisi(s) dans le groupe comprenant le fluor-18, le brome-76, l'iode-123, l'iode-131,
• M₁ est un contre-anion choisi parmi les cations, de préférence dans le groupe des cations monovalents.

2. Utilisation selon la revendication 1, **caractérisé en ce que** le composé répond à la formule 1a ou 1b

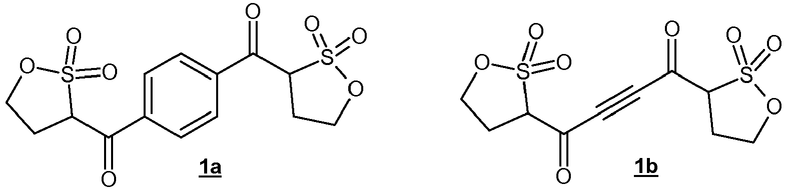

3. Utilisation selon la revendication 1 ou 2, **caractérisée en ce que** ladite macromolécule est choisie dans le groupe comprenant les peptides, les protéines, les oligonucléotides, les polynucléotides.

4. Composé de formules **6a** ou **6b** suivante : 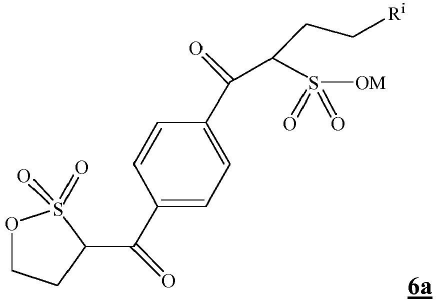 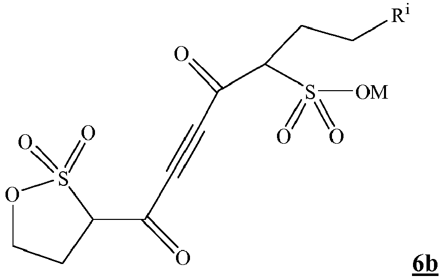 , **caractérisé en ce que**
• Rⁱ est unradionucléide nucléophile, de préférence choisi dans le groupe comprenant le fluor-18, le brome-76, l'iode-123, l'iode-131,
• M est un contre-anion choisi parmi les cations, de préférence les cations monovalents.

5. Composé de formule **7a** ou **7b** suivante : 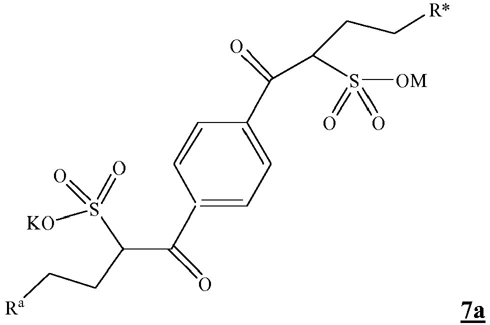 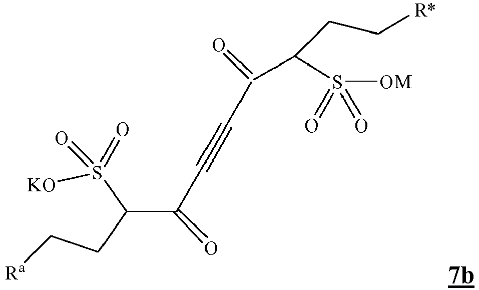 formules dans lesquelles, R* est un radionucléide, R^{a} est une macromolecule, M est un contre-anion choisi parmi les cations, de préférence les cations monovalents.

6. Composé selon la revendication 5, dans lequel R* est choisi parmi le fluor-18, le brome-76, l'iode-123, l'iode-131.

7. Composé selon la revendication 5 ou 6, dans lequel R^{a} est choisi parmi les peptides, les protéines, les oligonucléotides, les polynucléotides.

8. Procédé d'obtention des composés selon l'une des revendications 5 à 7, **caractérisé en ce qu'**il comprend les étapes suivantes :
a. mise en oeuvre ou synthèse d'une polysultone, comprenant au moins deux sultones reliées l'une à l'autre par au moins un espaceur et dont la formule (1) est la suivante: 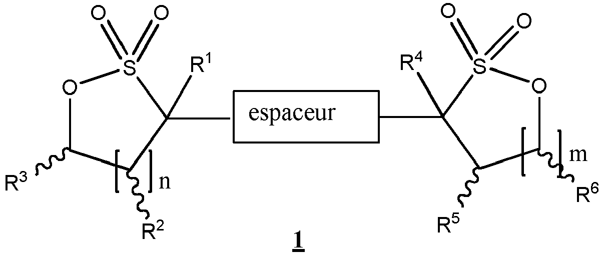 dans laquelle
- les groupements R¹ à R⁶, identiques ou différents, correspondent à l'hydrogène ou un alkyle, un aryle, un arylalkyle, un alkylaryle, un acyle, un cycloalkyle; R³ à R⁶ correspondant de préférence à l'hydrogène ;
- n, m est un entier, de préférence compris entre 0 et 2 ;
- l'espaceur est une structure divalente, de préférence choisie parmi celles comportant un ou plusieurs groupements aromatiques, alkyles, alcènes, alcynes, éventuellement combinés entre eux ;
b. ouverture de l'une des sultones avec un radionucléide nucléophile conduisant à la formation d'un sulfonate ;
c. séparation entre la polysultone, précurseur et le sulfonate formé par différence de polarité ;
d. recueil du sulfonate formé ;
e. couplage du sulfonate formé avec au moins un second nucléophile par l'ouverture d'une seconde sultone, ledit second nucléophile étant choisi parmi les macro molécules ;
f. recueil du composé obtenu à l'étape e.

9. Médicament ou produit de diagnostic comprenant au moins un composé selon la revendication 5 à 7 ou obtenu par le procédé selon la revendication 8.

## Patentansprüche

1. Verwendung einer Verbindung zur Markierung von Makromolekülen mit einem nukleophilen Radionukleid, **dadurch gekennzeichnet, dass** die Verbindung die allgemeine Formel 1 oder 2 hat: 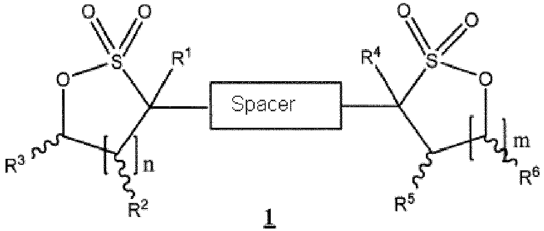 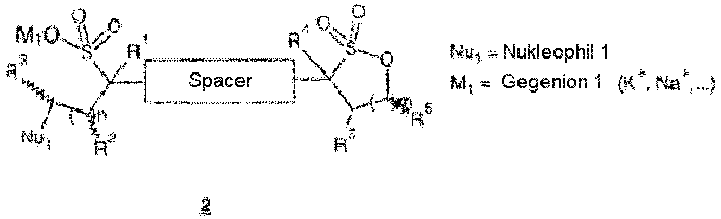 wobei in diesen Formeln:
* die Gruppierungen R¹ bis R⁶, die identisch oder unterschiedlich sind, dem Wasserstoff oder einem Alkyl, einem Aryl, einem Arylalkyl, einem Alkylaryl, einem Acyl, einem Cycloalkyl entsprechen, wobei R³ bis R⁶ vorzugsweise dem Wasserstoff entsprechen;
* n, m eine ganze Zahl vorzugsweise zwischen 0 und 2 ist;
* der Spacer eine divalente Struktur ist, die vorzugsweise unter jenen ausgewählt ist, die eine oder mehrere aromatische Gruppierungen, Alkyle, Alkene, Alkine, eventuell in Kombination untereinander, umfassen;
* Nu₁ ein nukleophiles Radionukleid R* ist, vorzugsweise ausgewählt in der Gruppe, umfassend das Fluor-18, das Brom-76, das Iod-123, das Iod 131,
* M₁ ein Gegenanion ist, das unter den Kationen, vorzugsweise in der Gruppe der monovalenten Kationen, ausgewählt ist.

2. Verwendung nach Anspruch 1, **dadurch gekennzeichnet, dass** die Verbindung der Formel 1a oder 1b entspricht:

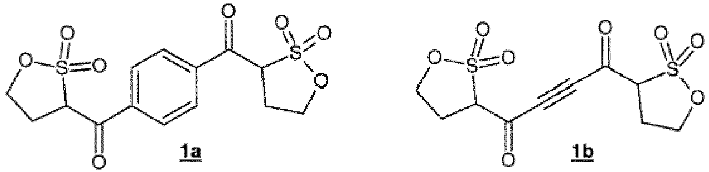

3. Verwendung nach Anspruch 1 oder 2, **dadurch gekennzeichnet, dass** das Makromolekül in der Gruppe, umfassend die Peptide, die Proteine, die Oligonukleotide, die Polynukleotide, ausgewählt ist.

4. Verbindung mit folgender Formel 6a oder 6b: 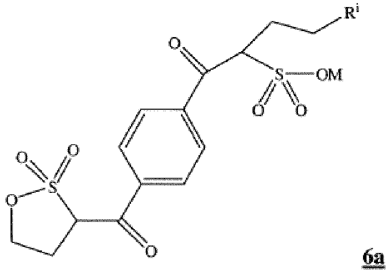 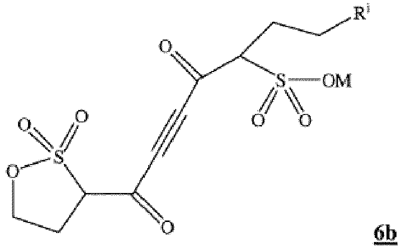 **dadurch gekennzeichnet, dass**
* Rⁱ ein nukleophiles Radionukleid ist, vorzugsweise ausgewählt in der Gruppe, umfassend das Fluor-18, das Brom-76, das Iod-123, das Iod-131,
* M ein Gegenanion ist, ausgewählt unter den Kationen, vorzugsweise den monovalenten Kationen.

5. Verbindung mit folgender Formel 7a oder 7b: 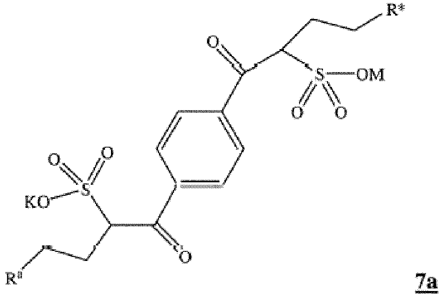 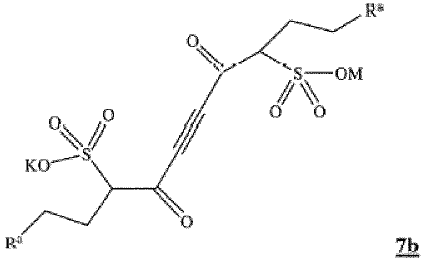 wobei in den Formeln R* ein Radionukleid ist, R^{a} ein Makromolekül ist, M ein Gegenanion ist, ausgewählt unter den Kationen, vorzugsweise den monovalenten Kationen.

6. Verbindung nach Anspruch 5, bei der R* unter dem Fluor-18, dem Brom-76, dem Iod-123, dem Iod-131 ausgewählt ist.

7. Verbindung nach Anspruch 5 oder 6, bei der R^{a} unter den Peptiden, den Proteinen, den Oligonukleotiden, den Polynukleotiden ausgewählt ist.

8. Verfahren zur Herstellung der Verbindungen nach einem der Ansprüche 5 bis 7, **dadurch gekennzeichnet, dass** es die folgenden Schritte umfasst:
a. Einsatz oder Synthese eines Polysultons, umfassend mindestens zwei Sultone, die miteinander durch mindestens einen Spacer verbunden sind, und deren Formel (1) folgende ist: 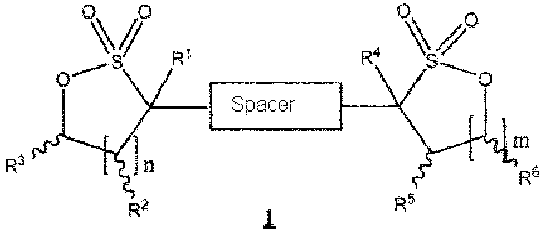 wobei:
* die Gruppierungen R¹ bis R⁶, die identisch oder unterschiedlich sind, dem Wasserstoff oder einem Alkyl, einem Aryl, einem Arylalkyl, einem Alkylaryl, einem Acyl, einem Cycloalkyl entsprechen, wobei R³ bis R⁶ vorzugsweise dem Wasserstoff entsprechen;
* n, m eine ganze Zahl vorzugsweise zwischen 0 und 2 ist;
* der Spacer eine divalente Struktur ist, die vorzugsweise unter jenen ausgewählt ist, die eine oder mehrere aromatische Gruppierungen, Alkyle, Alkene, Alkine, eventuell in Kombination untereinander, umfassen;
b. Öffnen eines der Sultone mit einem nukleophilen Radionukleid, was zur Bildung eines Sulfonats führt;
c. Trennung zwischen dem Polysulton, Vorläufer und dem durch Polaritätsunterschied gebildeten Sulfonat;
d. Aufnahme des gebildeten Sulfonats;
e. Kopplung des gebildeten Sulfonats mit mindestens einem zweiten Nukleophil durch das Öffnen eines zweiten Sultons, wobei das zweite Nukleophil unter den Makromolekülen ausgewählt ist;
f. Aufnahme der in Schritt e hergestellten Verbindung.

9. Medikament oder Diagnoseprodukt, umfassend mindestens eine Verbindung nach Anspruch 5 bis 7 oder erhalten durch das Verfahren nach Anspruch 8.

## Claims

1. Use of a compound for labelling macromolecules with a nucleophilic radionuclide, **characterized in that** said compound is of general formula 1 or 2: 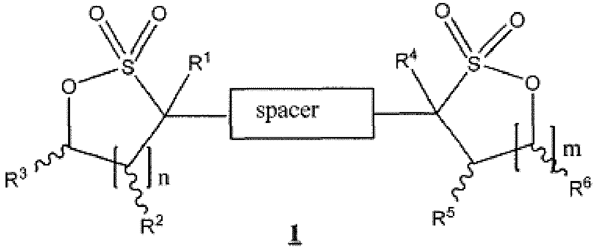 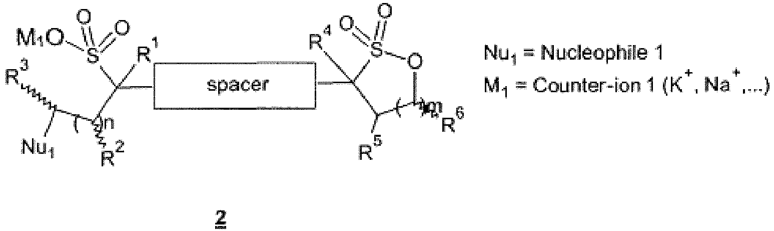 formulae in which
- the R¹ to R⁶ groups, identical or different, correspond to hydrogen or an alkyl, an aryl, an arylalkyl, an alkylaryl, an acyl, a cycloalkyl; R³ to R⁶ preferably corresponding to hydrogen;
- n, m is an integer, preferably comprised between 0 and 2;
- the spacer is a divalent structure, preferably selected from those comprising one or more aromatic, alkyl, alkene or alkyne groups optionally combined together;
- Nu₁ is a nucleophilic radionuclide R*, preferably selected from the group comprising fluorine-18, bromine-76, iodine-123, iodine-131,
- M₁ is a counter-anion selected from the cations, preferably from the group of the monovalent cations.

2. Use according to claim 1, **characterized in that** the compound is of formula **1a** or **1b**

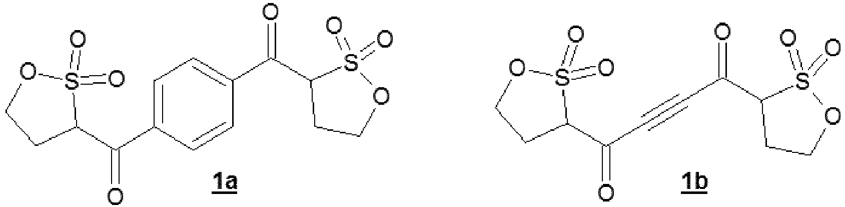

3. Use according to claim 1 or 2, **characterized in that** said macromolecule is selected from the group comprising peptides, proteins, oligonucleotides, polynucleotides.

4. Compound of the following formula **6a** or **6b:** 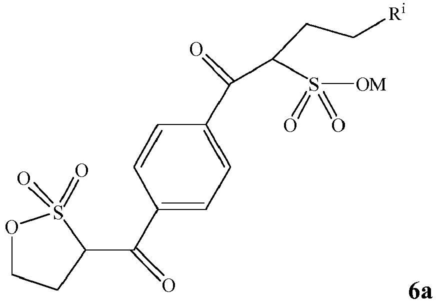 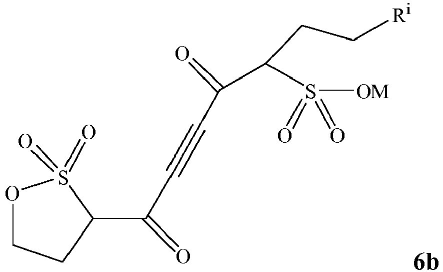 **characterized in that**
- Rⁱ is a nucleophilic radionuclide, preferably selected from the group comprising fluorine-18, bromine-76, iodine-123, iodine-131,
- M is a counter-anion selected from the cations, preferably from the group of the monovalent cations.

5. Compound of the following formula **7a** or **7b:** 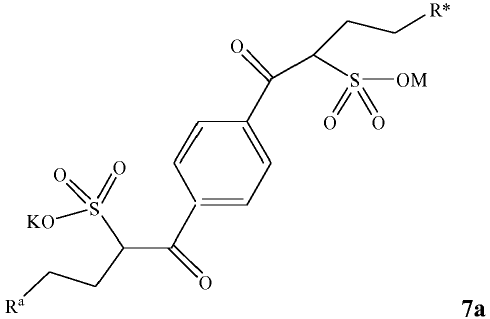 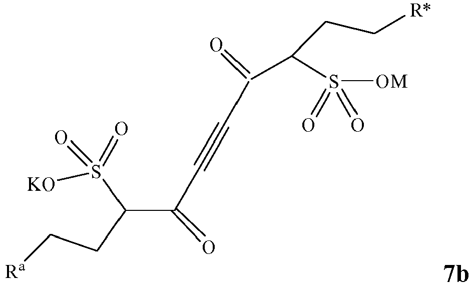 formulae in which, R* is a radionuclide, R^{a} is a macromolecule, M is a counter-anion selected from the cations, preferably from the group of the monovalent cations.

6. Compound according to claim 5, in which R* is chosen from fluorine-18, bromine-76, iodine-123, iodine-131.

7. Compound according to claim 5 or 6 in which R^{a} is chosen from peptides, proteins, oligonucleotides, polynucleotides.

8. Method for obtaining compounds according to one of claims 5 to 7, **characterized in that** it comprises the following stages:
a. Utilization or synthesis of a polysultone, comprising at least two sultones linked to each other by at least one spacer and having the following formula (1): 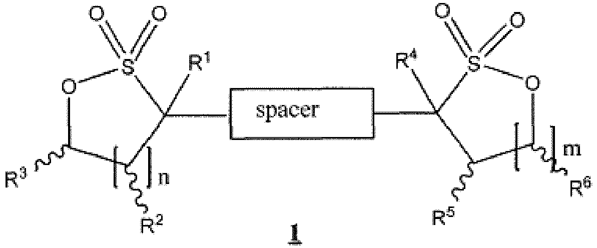 in which
- the R¹ to R⁶ groups, identical or different, correspond to hydrogen or an alkyl, an aryl, an arylalkyl, an alkylaryl, an acyl, a cycloalkyl; R³ to R⁶ preferably corresponding to hydrogen;
- n, m is an integer, preferably comprised between 0 and 2;
- the spacer is a divalent structure, preferably selected from those comprising one or more aromatic, alkyl, alkene or alkyne groups optionally combined together;
b. Opening of one of the sultones with a nucleophilic radionuclide leading to the formation of a sulphonate;
c. Separation of the polysultone precursor from the formed sulphonate by difference in polarity;
d. Collection of the formed sulphonate;
e. Coupling of the formed sulphonate with at least one second nucleophile by the opening of a second sultone, said second nucleophile being chosen from macromolecules;
f. Collection of the compound obtained in Stage e.

9. Drug or diagnosis product comprising at least one compound according to one of claims 5 to 7 or obtained by the method according to claim 8.
